# EUROPEAN PATENT SPECIFICATION

(11) **EP 2 051 749 B1**
(45) Date of publication and mention of the grant of the patent: **22.09.2010**
(21) Application number: 07785763.9
(22) Date of filing: 13.07.2007
(51) Int. Cl.: A61L 28/00, A61F 5/445, B32B 27/22

(54) **FILM PLASTICIZED WITH PLASTICIZER HAVING LOW VAPOUR PRESSURE**
MIT EINEM WEICHMACHER MIT NIEDRIGEM DAMPFDRUCK WEICHGEMACHTE FOLIE
FILM PLASTIFIÉ AVEC UN PLASTIFIANT AYANT UNE PRESSION DE VAPEUR FAIBLE

(30) Priority: 03.08.2006 DK 200601029
(43) Date of publication of application: 29.04.2009
(73) Proprietor: Coloplast A/S, 3050 Humlebæk (DK)
(72) Inventor: NIELSEN, Soeren Kirkegaard, 5600 Faaborg (DK)
(86) International application number: PCT/DK2007/050095
(87) International publication number: WO 2008/014789

(56) References cited:
- EP-B1- 0 703 762
- WO-A-03/020328
- GB-A- 2 083 762
- GB-A- 2 193 925
- GB-A- 2 201 372
- GB-A- 2 289 647
- US-A1- 2005 084 634

## Description

### Field of the invention

The present invention relates to a film suitable for use as an ostomy bag.

### Background

In connection with surgery for a number of diseases in the gastro-intestinal tract a consequence is, in many cases, that the colon, the ileum or the urethra has been exposed surgically and the patient is left with an abdominal stoma and the effluents or waste products of the body, which are conveyed through these organs, are discharged through the artificial orifice or opening and are collected in a collection bag, which is usually adhered to the skin by means of an adhesive wafer or plate having an inlet opening for accommodating the stoma. Also, in connection with a fistula, the patient will have to rely on an appliance to collect the bodily material emerging from such opening.

When replacing the ostomy bag, it would be advantageous for an ostomy-operated patient if the used ostomy bag could be w.c. disposable instead of having to go with the day refuse with odour problems as a consequence. However, in order to make such flushing of the used bag environmentally friendly it is required that the bag is biodegradable.

EP0703762 describes a so-called bag-in-bag solution. Here, the outer bag is similar to a conventional ostomy bag with charcoal filter. This outer bag provides the barrier towards odour. The inner bag, which is the flushable one, is biodegradable and or/water soluble. The two bags are separated before the inner bag is flushed out in the w.c.

GB2083762 describes a film made of PVOH coated with PVDC, vinyl chloride-vinylidene chloride-copolymer, atactic polypropylene, nitrocellulose, waxes, greases, silicones, or pressure-sensitive adhesives. The principle in a multi-layer laminate is that a biodegradable and/or water-soluble layer gives the main part of the mechanical strength, and a mechanical weak layer gives the main part of the barrier properties.

In the course of time several experiments have been made to prepare flushable ostomy bags, but the preparation of a foil that is both biodegradable and at the same time has the sufficient barrier towards faeces odour has not succeeded until now.

### Summary of the invention

The present invention discloses the construction of a biodegradable film that has a barrier towards faeces odour and with no cracking sounds. This is obtained by using a particular low vapour pressure plasticizer for the film material, followed by routine vacuum deposition of a glass or metal layer.

### Detailed Disclosure

A central aspect of the present invention relates to a film of biodegradable material plasticized with a polymeric plasticizer or another plasticizer of low vapour pressure, the film comprising on one or both surfaces a coating of glass or metal.

This film meets the following requirements:
1) It is biodegradable, i.e. 90 percentage of the weight of the film is degraded after six months (as determined by ISO 14852);
2) It is impervious to water and faeces odour for at least 10 hours;
3) It is not more stiff or more crackling than films presently used for ostomy bags.

In preferred embodiments, the film also is heat-sealable and transparent.

Hereby a dilemma in the art is solved: Glass and metal can only be vapour-deposited on polymers that do not contain plasticizers, as the plasticizer evaporates during the vacuum process. By selecting plasticizers with a low vapour pressure, the problems due to evaporation are solved. The present invention thus provides an ostomy bag comprising a film of biodegradable material plasticized with a plasticizer having a low vapour pressure, the film comprising on at least one surface a coating of glass or metal

It is preferred that the vapour pressure of the plasticizer is below 10⁻⁴ mm Hg at 25°C. The vapour pressure depends on the pressure used during the glass deposition process. In one embodiment the plasticizer is a plasticizer with a vapour pressure below 10⁻⁷ mm Hg at 25°C.

The table shows the approximate vapour pressure for some common monomeric plasticizers. It is seen that it is also possible to choose a common plasticizer with a low vapour pressure. F.x. Benzoflex 50 supplied from Velsicol Chemical, which is compatible with PLA and has a vapor pressure of 2,29x10⁻⁷ mmHg at 25°C.

| **Vapor pressure range** **(mm Hg at 25°C)** | **Chemical name** |
|---|---|
| 10⁻³ | Dimethyl phthalate, Diethyl phthalate, Diisobuthyl phthalate |
| 10⁻⁴ | Diallyl phthalate |
| 10⁻⁵ | Di-n-butyl phthalate, Dihexyl phthalate |
| 10⁻⁶ | Butyl benzyl phthalate, Diisooctyl phthalate, Dibutyl sebacate, Di-n-octyl phthalate, Acetyl tributyl citrate, Di-(2-ethylhexyl) azelate, Di-(2-ethylhexyl) sabacate |
| 10⁻⁷ | Di-(2-ethylhexyl) adipate, Di-n-octyl adipate, Tricresyl phosphate, Diisononyl phthalate, Diisodecyl phthalate, Dinonyl phthalate |
| 10⁻⁸ | Di-(2-ethylhexyl) phthalate |
| 10⁻⁹ | Diundecyl phthalate |
| 10⁻¹⁰ | |
| 10⁻¹¹ | Tri-(2-ethylhexyl) trimellitate, Ditridecyl phthalate |

George Wypych, Handbook of Plasticizers, p. 605, ChemTec Publishing, Toronto 2004 .

In one embodiment the plasticizer is a polymer itself, for example a polyester plasticizer. In another embodiment, the plasticizer is a monomeric plasticizer.

The amount of plasticizer used is 5-50%, such as 10-30% depending on how soft the material (the film) should be.

Applying the plastizicer to the film material is well known to the skilled person. In one method, the plasticizer is compounded into the polymer in a molten state by means of an extruder or a mixer at elevated temperature. Alternatively the polymer is dissolved and mixed with plasticizer. Subsequently a film can be casted from the mixture.

The purpose of the coating of the at least one side of the film is to make the film odour, water an moisture impermeable. Thus, the coating is a continuous layer of a glass or metal. It is well known that a layer of SiOx or aluminum improves the barrier properties of a PET film (see figure 1 from: Barrier Technologies, AIMCAL Fall Technical Conference, Dr. B.M.Henry, University of Oxford, 2005). Similar barrier improvements are seen by coating a biodegradable film with SiOx or aluminum.

Vacuum deposition of glass or aluminum to the film material is well known to the skilled person. An example of such procedure is described in "Barrier properties of SiOx-coated polymers: multi-layer modelling and effects of mechanical folding", M.S. Hedenqvist, Surface and Coatings Technology 172 (2003) 7-12.

Vacuum deposition, or co-extruding of the film material with other barrier coatings is also conceivable. That includes other metal oxides or DLC (diamond like carbon) and organic polymeric surfaces optionally containing Si, Cl or F.

In one aspect of the invention the biodegradable material is degraded 90% (of weight) after six months. It is, however, preferred that the biodegradable material is degraded 100% after six months, allowing for 10% of the weight of the film to be non-degradable material (e.g. the coating of glass or metal). In a preferred embodiment, the biodegradable material is selected among biodegradable polyesters in general. Particularly preferred is the biodegradable material selected from the group consisting of PVOH (polyvinyl alcohol), Starch, PLA (polylatic acid), PHB (polyhydroxybutyrate), and Polycaprolactone. Examples of such films are Mater-Bi, Eco-flex and PVOH.

In the present invention, a "film" is understood as a thin, planar sheet with a thickness of about 10-100 micrometer. Typically, the dimensions of the film for use as an ostomy bag (height x width) are in the range of 126x200mm.

This solution is advantageous, as you do not need to separate bags before flushing the inner bag and keeping the (somewhat soiled) outer bag.

The film can also be used in other contexts, where biodegradability/solubility shall be combined with a high barrier, for instance for urine bags, but also in completely other connections where a product is to be sealed, for instance for packaging of foodstuffs or where a controlled release is desired, i.e. the packaging degrades/dissolves, whereby the contents is released.

### Examples

### Example 1: Plasticizing PLA

A plasticized PLA film is made by using a twin-screw extruder (ThermoPrism Eurolab) with a 16 mm screw diameter and 30 UD. The processing temperature is set at 125 - 180°C from feed inlet to the die. The extruder is equipped with a film sheet die and a film take-off unit.

PLA pellets (Biomer L9000) are gravimetrically fed and the plasticizer (Benzoflex 50 [50:50 diethylene glycol dibenzoate : dipropylene glycol dibenzoate]) volumetrically added simultaneously further down the extruder barrel, where the referred PLA pellets are melted. This results in an extrusion of a 50µm thick and 10cm wide film. The composition of the film is 80% PLA and 20% Benzoflex.

### Example 2: Plasma deposition of SiOx

A 50µm plasticized film sample (80% PLA, 20% Benzoflex) is coated with SiOx by the use of plasma deposition. The plasticized film sample is placed in a reactor and a mixture of hexamethyldisiloxane (HMDSO) and oxygen is added in the reactor producing the SiOx compound, which coats the film sample. This deposition is performed with the parameter settings shown in the table below:

| Type of plasma | Process gas / monomer | Power (W) | Flow rate (sccm) | Pressure (mtorr) | Deposition time (min.) | Frequency (kHz) |
|---|---|---|---|---|---|---|
| SiOx | HMDSO + O₂ 1:10 | 150 | 2.5:25 | 75 | 1 | 140 |

### Example 3: Crackling of bags

It is important to have a soft film, as cracking of the film is unfortunate. In order to determine the crackling of bags, three blinded people were asked to determine the cracling on a scale from 1 = no crackling to 5 = very crackling.

The table below shows three people's evaluation of the crackling of thirteen different films. Films E and F are the type of films that are used for conventional ostomy bags. The films E and F are the golden standard for how little crackling is acceptable.

Film I is a non-plasticized biodegradable film. Films J, K, L and M are plasticized biodegradable films. Film I is very crackling, whereas the plasticized films are less crackling. All these films (A-M) are without glass barrier layer, and the glass layer (N) is not expected to add considerably to the crackling.

Films G and H are water-soluble PVOH films. Film H is embossed, i.e. the surface has a pattern structure which seems to lower the crackling.

Films A, B, C and D are common films.

| | Type | Person I | Person II | Person III | Average | STDEV |
|---|---|---|---|---|---|---|
| A: PET 50my | | 5 | 5 | 5 | 5,0 | 0,0 |
| B: LDPE 40my | | 3 | 4 | 3 | 3,3 | 0,6 |
| C: EMA 70my | | 1 | 2 | 1 | 1,3 | 0,6 |
| D: PU 60my | | 1 | 3 | 1 | 1,7 | 1,2 |
| E: Saranex 630 80my | current ostomy bag | 2 | 3 | 3 | 2,7 | 0,6 |
| F: Saranex 114 60my | current ostomy bag | 4 | 4 | 3 | 3,7 | 0,6 |
| G: PVOH 80my | | 4 | 5 | 4 | 4,3 | 0,6 |
| H: PVOH (embossed) (80my) | | 3 | 3 | 2 | 2,7 | 0,6 |
| I: PLA 20my | Non plasticized biodegradable film | 5 | 5 | 5 | 5,0 | 0,0 |
| J: PLA + 20% Benzoflex 50my | plasticized biodegradable film | 4 | 4 | 4 | 4,0 | 0,0 |
| K: Mater-Bi 90my | plasticized biodegradable film | 4 | 4 | 4 | 4,0 | 0,0 |
| L: Mater-Bi 20my | plasticized biodegradable film | 3 | 2 | 1 | 2,0 | 1,0 |
| M: Ecoflex 40my | plasticized biodegradable film | 2 | 4 | 2 | 2,7 | 1,2 |
| N: PLA + 20% Benzoflex 50my, coated with SiOx | plasticized coated biodegradable film | 4 | 4 | 4 | 4 | 0 |

It can be observed from the above table that the plasticized PLA film (J) is about as crackling as a typical film for ostomy bags (Saranex 114, F). It can also be observed that a SiOx-coating is not expected to increase the crackling noticeably (N).

### Example 4: Odour of bags

To determine the odour coming through the bag, we conducted the following test. Bags of each material were made out of two pieces of film (120 x 120 mm). The films are heat sealed at three sides. The last side is welded after adding 1 ml of a 0.2 % aqueous skatol solution. The bags are placed in separate closed glass jars and kept at 32°C. Three blinded people at three different time points evaluate the smell.

The odour scale was 0 = no smell, 3 = strong smell

| | | 1,5 h | 4 h | 24 h |
|---|---|---|---|---|
| L: Mater-Bi 20my | plasticized biodegradable film | 1 | 2 | 3 |
| Mater-Bi 80my | | 0 | 2 | 3 |
| Ecoflex | | 1 | 3 | 3 |
| E: Saranex 630 | current ostomy bag | 0 | 0 | 0 |
| F: Saranex | current ostomy bag | 0 | 0 | 0 |
| B: LDPE 40my | | 2 | 3 | 3 |
| G: PVOH | | 2 | 3 | 3 |

It is seen that the PVDC containing Saranex films are much better than anything else and set the goal for the odour barrier. Neither a common non-biodegradable film like LDPE nor any biodegradable films provide a sufficient barrier towards ostomy gases. It is expected that the plasticized biodegradable films with deposited glass layer provide a sufficient barrier towards ostomy gases, as determined by this skatol test.

## Claims

1. Film of biodegradable material plasticized with a plasticizer having a vapour pressure below 10⁻⁴ mm Hg at 25°C, the film comprising on at least one surface a coating of vacuum deposited glass or metal.

2. Film according to claim 1, wherein the vapour pressure of the plasticizer is below 10⁻⁷ mm Hg at 25°C.

3. Film according to any of the previous claims, wherein the plasticizer is a polymeric plasticizer.

4. Film according to claim 3, wherein the polymeric plasticizer is a polymeric polyester plasticizer.

5. Film according to any of the previous claims, wherein the plasticizer is a monomeric plasticizer with a vapour pressure below 10⁻⁴ mm Hg at 25°C.

6. Film according to any of the previous claims, wherein the plasticizer is a monomeric plasticizer with a vapour pressure below 10⁻⁷ mm Hg at 25°C.

7. Film according to any of the previous claims, wherein the film comprises a coating of glass or metal on both surfaces.

8. Film according to any of the previous claims, wherein the biodegradable material is degraded 90% (of weight) after six months.

9. Film according to any of the previous claims, wherein the biodegradable material is selected from the group consisting of PVOH (polyvinyl alcohol), Starch, PLA (polylatic acid), PHB (polyhydroxybutyrate), and Polycaprolactone.

10. Film according to any of the previous claims, wherein the film has a thickness of 10 to 100 micrometer.

11. An ostomy bag formed of a film according to any of the preceding claims.

12. A urine bag formed of a film according to any of claims 1-10.

## Patentansprüche

1. Folie aus einem biologisch abbaubaren Material, das mit einem Weichmacher mit einem Dampfdruck bei 25 °C von weniger als 10⁻⁴ mmHg weichgemacht ist, wobei die Folie auf mindestens einer Oberfläche eine Beschichtung aus im Vakuum aufgedampftem Glas oder Metall aufweist.

2. Folie nach Anspruch 1, bei welcher der Dampfdruck des Weichmachers bei 25 °C weniger als 10⁻⁷ mmHg beträgt.

3. Folie nach einem oder mehreren der vorhergehenden Ansprüche, bei welcher der Weichmacher ein polymerer Weichmacher ist.

4. Folie nach Anspruch 3, bei welcher der polymere Weichmacher ein polymerer Polyester-Weichmacher ist.

5. Folie nach einem oder mehreren der vorhergehenden Ansprüche, bei welcher der Weichmacher ein monomerer Weichmacher mit einem Dampfdruck bei 25 °C von weniger als 10⁻⁴ mmHg ist.

6. Folie nach einem oder mehreren der vorhergehenden Ansprüche, bei welcher der Weichmacher ein monomerer Weichmacher mit einem Dampfdruck bei 25 °C von weniger als 10⁻⁷ mHg ist.

7. Folie nach einem oder mehreren der vorhergehenden Ansprüche, wobei die Folie auf beiden Oberflächen eine Beschichtung aus Glas oder Metall aufweist.

8. Folie nach einem oder mehreren der vorhergehenden Ansprüche, bei der das biologisch abbaubare Material nach sechs Monaten zu 90 % (gewichtsbezogen) abgebaut ist.

9. Folie nach einem oder mehreren der vorhergehenden Ansprüche, bei der das biologisch abbaubare Material aus der Gruppe ausgewählt ist, die besteht aus PVOH (Polyvinylalkohol), Stärke, PLA (Polymilchsäure), PHP (Polyhydroxybutyrat) und Polycaprolacton.

10. Folie nach einem oder mehreren der vorhergehenden Ansprüche, wobei die Folie eine Dicke von 10 bis 100 Mikrometer besitzt.

11. Stomabeutel, der aus einer Folie nach einem oder mehreren der vorhergehenden Ansprüche hergestellt ist.

12. Urinbeutel, der aus einer Folie nach einem oder mehreren der Ansprüche 1 bis 10 hergestellt ist.

## Revendications

1. Film de matériau biodégradable plastifié avec un plastifiant ayant une pression de vapeur inférieure à 10⁻⁴ mm Hg à 25°C, le film comprenant sur au moins une surface un revêtement de verre ou de métal déposé sous vide.

2. Film selon la revendication 1, dans lequel la pression de vapeur du plastifiant est inférieure à 10⁻⁷ mm Hg à 25°C.

3. Film selon l'une quelconque des revendications précédentes, dans lequel le plastifiant est un plastifiant polymère.

4. Film selon la revendication 3, dans lequel le plastifiant polymère est un plastifiant de polyester polymère.

5. Film selon l'une quelconque des revendications précédentes, dans lequel le plastifiant est un plastifiant monomère avec une pression de vapeur inférieure à 10⁻⁴ mm Hg à 25°C.

6. Film selon l'une quelconque des revendications précédentes, dans lequel le plastifiant est un plastifiant monomère avec une pression de vapeur inférieure à 10⁻⁷ mm Hg à 25°C.

7. Film selon l'une quelconque des revendications précédentes, dans lequel le film comprend un revêtement de verre ou de métal sur les deux surfaces.

8. Film selon l'une quelconque des revendications précédentes, dans lequel le matériau biodégradable est dégradé à 90 % (en poids) après six mois.

9. Film selon l'une quelconque des revendications précédentes, dans lequel le matériau biodégradable est choisi dans le groupe comprenant du PVAL (alcool polyvinylique), de l'amidon, de l'APL (acide polylactique), du PHB (polyhydroxybutyrate) et du polycaprolactone.

10. Film selon l'une quelconque des revendications précédentes, dans lequel le film possède une épaisseur de 10 à 100 micromètres.

11. Poche pour stomie formée d'un film selon l'une quelconque des revendications précédentes.

12. Poche à urine formée d'un film selon l'une des revendications 1 à 10.
